# EUROPÄISCHE PATENTSCHRIFT

(11) **EP 1 603 682 B1**
(45) Veröffentlichungstag und Bekanntmachung des Hinweises auf die Patenterteilung: **13.06.2007**
(21) Anmeldenummer: 04721856.5
(22) Anmeldetag: 19.03.2004
(51) Int. Cl.: B05B 1/18, B05B 15/00, B05B 1/34

(54) **DUSCHE MIT EINER BELEUCHTUNGSEINRICHTUNG**
SHOWER COMPRISING A LIGHTING DEVICE
DOUCHE POURVUE D'UN DISPOSITIF D'ECLAIRAGE

(30) Priorität: 19.03.2003 DE 10312866
(43) Veröffentlichungstag der Anmeldung: 14.12.2005
(73) Patentinhaber: Hansgrohe AG, 77761 Schiltach (DE)
(72) Erfinder: GROSS, Jürgen, 77790 Steinach (DE)
(74) Vertreter: Ruff, Michael
(86) Internationale Anmeldenummer: PCT/EP2004/002869
(87) Internationale Veröffentlichungsnummer: WO 2004/082846

(56) Entgegenhaltungen:
- WO-A-91/12896
- DE-U- 20 101 460
- DE-U- 20 121 306
- US-A- 6 021 960
- US-A- 6 126 290
- US-A1- 2002 158 153
- US-B1- 6 439 472

## Beschreibung

Die Erfindung geht aus von einer Dusche mit einem Brausegehäuse, einem Wassereinlassanschluss und einer Strahlscheibe, aus der das Wasser in Form einiger oder vieler Strahlen austritt.

Es ist seit langem bekannt, dass man zur Verbesserung des Wohlbefindens beim Duschen und auch aus medizinischen Gründen die Arten der Strahlen, die aus einer Brause kommen, vielfältig variieren kann. Hier gibt es Massagestrahlen, weiche belüftete Strahlen, harte Strahlen, die alle auch in Kombination und mit sich automatisch ändernden Umstellern verabreicht werden können, die zwischen den einzelnen Strahlarten umschalten.

Auch die Variation der Temperatur ist als Mittel zur Gesundheitsverbesserung und als Mittel zum Verbessern des Wohlfühlens bekannt.

Man hat nun auch erkannt, dass auch das Licht einen Einfluss auf das Wohlbefinden von Personen haben kann.

So ist es bekannt, in Duschkabinen variable Farblichteffekte zu erzeugen. Es sind auch Brausen mit Beleuchtungseinrichtungen bekannt. Allerdings ist deren Beleuchtungseffekt schwach, oder sie sind sehr groß, insbesondere hoch bauend.

Es ist bereits ein Brausekopf mit einer Beleuchtungseinrichtung bekannt (US 2002/0158153), bei der die Beleuchtungseinrichtung Lichtleiter aufweist, die in den Strahlaustrittsöffnungen münden. Dadurch werden die aus den Strahlaustrittsöffnungen austretenden Strahlen beleuchtet.

Weiterhin bekannt ist eine Brause (DE 20101460), bei der in dem Brausekopf ein Wasserverteilerraum angeordnet ist, in dem Leuchtmittel vorhanden sind, die das Wasser in diesem Verteilerraum beleuchten.

Ebenfalls bekannt ist einer Brause mit eingebauter Leuchte (WO 91/12896), bei der in einem Brausekopfinnenraum innerhalb des mit Wasser gefüllten Raums eine Leuchte angeordnet ist, die von der im Hohlraum fließenden Flüssigkeit umspült wird. Diese Umspülung ist erforderlich, um die Lampe zu kühlen.

Weiterhin ist ein Brausekopf bekannt, der eine flache wasserdurchströmte Kammer aufweist, deren Rückseite durch eine transparente Scheibe abgeschlossen ist. Dieser Brausekopf wird vor einer Vertiefung in einer Wand angebracht, wobei in der Vertiefung der Wand eine Lampe angeordnet ist EP 1239089 A2).

Der Erfindung liegt die Aufgabe zu Grunde, Brausen zu schaffen, mit denen es möglich ist, beim Duschen solche variable Lichteffekte zu erzeugen, die ein optisch ansprechendes Duschbild bieten und vielfältig einsetzbar sind.

Zur Lösung dieser Aufgabe schlägt die Erfindung eine Brause mit den im Anspruch 1 genannten Merkmalen vor. Weiterbildungen der Erfindung sind Gegenstand von Unteransprüchen.

Die Beleuchtungseinrichtung erzeugt eine Lichtstrahlung, die daher aus der Brause selbst kommt. Sie ist in die gleiche Richtung gerichtet wie die Brausestrahlen, und für den Benutzer ist dann, wenn die Brause nicht in Betrieb ist, kein Unterschied zu einer normalen Brause, das heißt einer Brause ohne Beleuchtungseinrichtung, erkennbar. Es kann erfindungsgemäß in Weiterbildung vorgesehen sein, dass die Beleuchtungseinrichtung nur dann eingeschaltet ist, wenn die Brause auch im Betrieb ist. Dadurch, dass das Licht aus der Strahlaustrittsscheibe austritt, werden die Wasserstrahlen, die aus den Austrittsöffnungen ja noch als einzelne Strahlen austreten, praktisch von innen beleuchtet, was einen optisch ansprechenden Eindruck ergibt.

Es ist ein interessantes Bild, wenn das Licht beispielsweise nur oder überwiegend aus den Strahlaustrittsöffnungen austritt. Dabei ist es denkbar, die Strahlscheibe so zu gestalten, dass sie lichtundurchlässig ist, was dazu führt, dass das Licht nur aus den Öffnungen austritt, durch die auch das Wasser nach außen gelangt.

Es ist aber jedenfalls möglich und wird von der Erfindung vorgeschlagen, dass das Licht aus größeren Bereichen austritt, die beispielsweise nicht zusammenhängend sind. Unter größer ist dabei eine Größe zu verstehen, die größer als die Strahlaustrittsöffnung ist.

In nochmaliger Weiterbildung der Erfindung kann auch vorgesehen sein, dass das Licht im wesentlichen aus der ganzen Fläche der Strahlscheibe austritt. Auch hier kann vorgesehen sein, dass das Licht aus den Strahlaustrittsöffnungen stärker austritt, was beispielsweise dadurch erreicht werden kann, dass die Strahlaustrittsscheibe durchscheinend und damit das Licht etwas dämpfend ausgebildet ist.

Erfindungsgemäß kann in dem Brausegehäuse auch noch eine zusätzliche Lampe angeordnet sein, vorzugsweise zentral bzw. mittig, oder auch als Kranz an der Außenseite.

In nochmaliger Verbesserung der optischen Erscheinung, die bei der Benutzung der Brause auftritt, kann vorgesehen sein, dass hinter der Strahlscheibe eine Kammer ausgebildet ist, in die der Wassereinlass führt. Diese Kammer ist beim Betrieb der Brause dann vollständig mit Wasser gefüllt. Erfindungsgemäß kann nun die Beleuchtungseinrichtung hinter dieser Kammer angeordnet sein, so dass das Licht der Beleuchtungseinrichtung durch die Kammer hindurch zur Strahlaustrittsscheibe und von dort nach außen gelangt.

Zwischen der mit Wasser gefüllten Kammer und der Beleuchtungseinrichtung kann vorzugsweise eine durchsichtige bis durchscheinende Wand angeordnet sein, die je nach dem Grad der Durchsichtigkeit und einer möglichen Musterung unterschiedliche Arten von Lichterscheinungen ermöglicht.

Die Dicke der Kammer, das heißt die Abmessung der Kammer senkrecht zur Fläche der Strahlscheibe, kann ebenfalls dazu herangezogen werden, die Lichterscheinungen zu beeinflussen. Die Erfindung schlägt vor, dass für eine möglichst gleichmäßige Lichterscheinung über die Quererstreckung der Strahlscheibe die Dicke der Kammer überall gleich ist.

Die Querabmessung der Kammer kann in Weiterbildungen der Erfindung genauso groß sein wie die Querabmessung der Strahlscheibe. Mit anderen Worten ist die Kammer überall hinter der Strahlscheibe angeordnet, und vorzugsweise auch überall gleich dick.

In Weiterbildung der Erfindung kann vorgesehen sein, dass die Strahlscheibe in zwei zueinander senkrecht stehenden Richtungen etwa gleich groß ist, also beispielsweise rund oder quadratisch.

Die Erfindung schlägt vor, die hinter der Strahlscheibe angeordnete mit Wasser gefüllte Kammer sehr flach auszubilden, beispielsweise mit einer Dicke, die 1 - 10 % des Durchmessers der Strahlscheibe entspricht. Auf Grund dieser sehr flachen Ausbildung der Kammer treten Strömungen, Verwirbelungen oder dergleichen hinter der Strahlscheibe auf, die sehr ansprechende Lichterscheinungen nach sich ziehen.

Diese Erscheinung wird noch verstärkt, wenn in Weiterbildung der Erfindung die Kammer und/oder die Strahlscheibe so angeordnet und ausgebildet ist, dass in der Kammer das Entstehen von Wasserverwirbelungen und / oder radialen Strömungen begünstigt wird.

Darüber hinaus ist das Volumen der Kammer, die auf Grund von Kapillarwirkung nach dem Abschalten der Brause möglicherweise mit Wasser gefüllt bleibt, sehr klein. Dadurch verringert sich die Gefahr der Verkeimung bei längerem Nichtgebrauch.
Erfindungsgemäß kann vorgesehen sein, dass die Beleuchtungseinrichtung zum Erzeugen des Lichts LEDs enthält. Diese können auf engem Raum angeordnet werden und sind in der Lage, farbiges Licht abzugeben. Nach einem weiteren Merkmal der Erfindung kann nämlich die Farbe als weiteres Gestaltungselement verwendet werden. Durch entsprechende Ansteuerung der LED's können beliebige Variationen erzeugt werden.

Es hat sich gezeigt, dass eine radiale Anordnung der LED's eine besonders gleichmäßige Abgabe des Farblichts ergibt. Deshalb und um die Brause flach ausbilden zu können, kann erfindungsgemäß vorgesehen sein, dass die Beleuchtungseinrichtung radial abstrahlende Lichtquellen aufweist, also Lichtquellen, die Licht in einer Richtung parallel zur Strahlscheibe abgeben. Dieses Licht kann dann durch eine Umwelteinrichtung, einen Spiegel oder dergleichen, zur Strahlscheibe gelenkt werden.

Die radiale Anordnung in Zusammenhang mit der Umlenkeinrichtung ergibt eine flache Bauweise. Deshalb kann die Brause nicht nur als Kopfbrause ausgebildet sein, was sich in erster Linie anbietet, sondern auch als Seitenbrause beispielsweise in der Wand einer Duschkabine, oder auch als Handbrause.

Weitere Merkmale, Einzelheiten und Vorzüge der Erfindung ergeben sich aus den Patentansprüchen und der Zusammenfassung, deren beider Wortlaut durch Bezugnahme zum Inhalt der Beschreibung gemacht wird, der folgenden Beschreibung bevorzugter Ausführungsformen sowie anhand der Zeichnung. Hierbei zeigen:
- Figur 1: einen Querschnitt durch ein erstes Ausführungsbeispiel einer Brause nach der Erfindung;
- Figur 2: einen Querschnitt durch eine zweite Ausführungsform einer Brause nach Erfindung;
- Figur 3: einen Querschnitt durch eine dritte Ausführungsform der Brause nach der Erfindung.

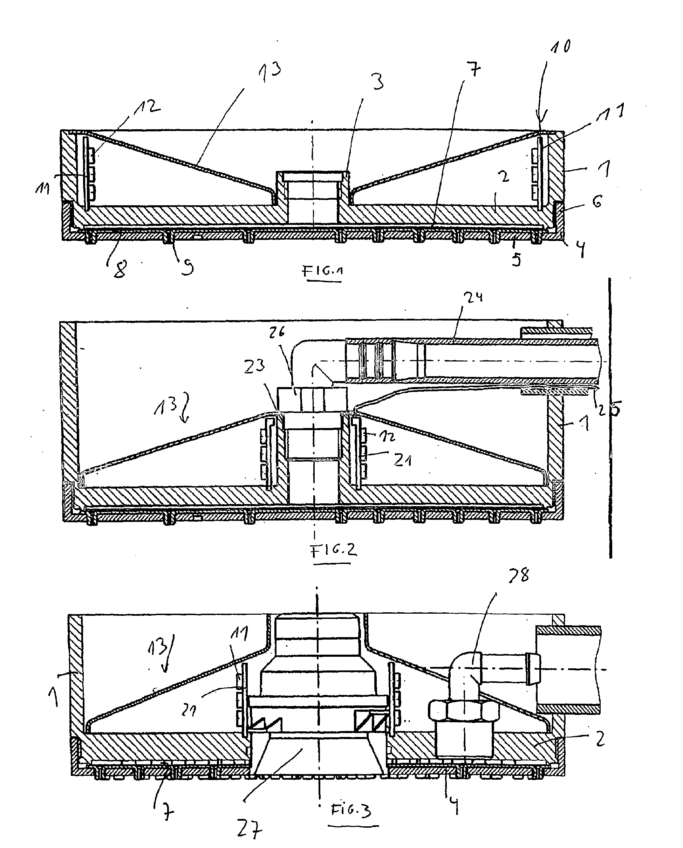

Figur 1 zeigt einen Querschnitt durch eine schematische dargestellte Brause. Die Brause enthält ein Brausegehäuse 1, das die Form eines flachen Zylinders mit einem Boden 2 aufweist. Mittig im Boden ist von der Rückseite, das heißt in Figur 1 von oben, ein Anschluss 3 eingesetzt. Dieser Anschluss 3 dient zum Anschließen einer Zuleitung.

Vor dem Boden 2 des Brausegehäuses 1 ist eine Strahlscheibe 4 angeordnet, die ebenfalls die Form einer Platte 5 mit einem zylindrischen Rand 6 aufweist. Mit diesem Rand 6 ist die Strahlscheibe 4 vor der Brause 1 so angebracht, dass zwischen dem Boden 2 des Gehäuses 1 und der Strahlscheibe ein flacher eine Kammer 7 bildender Zwischenraum bleibt. Zwischen dem zylindrischen Rand 6 und dem Boden 2 des Brausegehäuses 1 kann eine Abdichtung vorhanden sein, die aber auch durch eine aus elastischem Material bestehende Platte 8 gebildet sein kann. Die Platte 8 weist angeformte Düsen 9 auf, die durch entsprechende Öffnungen der Strahlscheibe 4 hindurch gehen. Die Platte 8 besteht aus durchsichtigem bzw. durchscheinendem Material. Auch der Boden 2 des Brausegehäuses 1 besteht aus durchsichtigem oder durchscheinendem Material.

Der Leitungsanschluss 3 führt direkt in die Kammer 7. Das in die Kammer 7 durch den Anschluss 3 gelangende Wasser verteilt sich in der gesamten Kammer 7 und strömt dann aus den Strahlaustrittsöffnungen 9 aus. Für die hier vorliegende Problematik ist es nicht erforderlich, dass die Strahlaustrittsöffnungen 9 an der Platte 8 aus elastischem Material angeformt sind. Es könnte auch eine Strahlscheibe 4 vorhanden sein, bei der einfache durchgehende Öffnungen als starre Austrittsöffnungen vorhanden sind.

Oberhalb des Bodens 1 des Brausegehäuses ist an dem Rand eine Beleuchtungseinrichtung 10 vorhanden, die an einer Platte 11 angeordnet ist, die sich in einzelnen Teilen oder durchgehend längs des Innenumfangs des Brausegehäuses 1 erstreckt. Die Platte 11 enthält drei übereinander liegende Reihen von LED's 12, die beispielsweise abwechselnde Farben haben können. Die LED's strahlen von der Platte 11 radial nach innen, das heißt in Richtung auf die Symmetrieachse der Anordnung.

Oberhalb des Bodens 2 des Brausegehäuses 1 ist eine Umlenkeinrichtung 13 angeordnet, die von einem flachen Kegel gebildet wird, dessen Kegelspitze zur Vorderseite des Brausegehäuses 1 gerichtet ist. Dort enthält die Umlenkeinrichtung 13 eine zentrale Öffnung, die dazu dient, die Umlenkeinrichtung an dem Anschluss 3 für das einzulassende Wasser zu zentrieren und festzulegen.

Wenn die Beleuchtungseinrichtung 10 eingeschaltet ist, so wird das von den LEDs 12 ausgehende Licht an der Umlenkeinrichtung 13 so umgelenkt, dass die Lichtstrahlen senkrecht auf den Boden 2 des Gehäuses treffen, durch diesen hindurch gehen und dann durch die Strahlscheibe 4 austreten. Je nach dem Material der Strahlscheibe können die Strahlen dabei durch die ganze Strahlscheibe oder nur durch die an sich ja offenen Austrittsöffnungen austreten. Wenn die Lichtstrahlen durch die Strahlaustrittsöffnungen austreten, so pflanzen sie sich in den Wasserstrahlen selbst fort, was ein ansprechendes Strahlbild ergibt.

Bei der Ausführungsform der Figur 2 ist der Leitungsanschluss 23 für die Zuleitung 24 etwas größer ausgebildet, und die Zuleitung 24 dargestellt. Sie tritt als Rohr durch den Rand 1 des Brausegehäuses 1 ein. An der gleichen Stelle wird auch eine Zuleitung 25 für elektrische Energie durchgeführt. Bei dieser Ausführungsform ist die Beleuchtungseinrichtung um den zentralen Leitungsanschluss 21 herum angeordnet. Sie enthielt hält wiederum eine Platte oder einen Zylinder 21, an dem die LEDs 12 angeordnet sind. Sie strahlen jetzt radial nach außen. Die Umlenkeinrichtung 13 enthält wieder einen flachen Kegel aus an der Unterseite reflektierendem Material. Der flache Kegel ist jetzt so orientiert, dass seine Spitze von der Strahlscheibe weg gerichtet ist. Er ist wiederum an dem Anschluss zentriert und durch eine Mutter 26 festgelegt.

Figur 3 zeigt eine Ausführungsform, bei der in der Mitte der Strahlaustrittsscheibe 4 eine größere Öffnung mit einer Lampe 27 vorhanden ist. Die Vorderseite der Lampe verläuft bündig mit der Vorderseite der Strahlscheibe 4. Wiederum ist eine Umlenkeinrichtung 13 ähnlich wie bei der Ausführungsform nach Figur 2 vorhanden. Die Beleuchtungseinrichtung entspricht wieder der der Figur 2. Die Zuleitung für das aus der Brause austretende Wasser ist jetzt exzentrisch angeordnet und mit Hilfe eines Nippels 28 von der Rückseite her in den durchsichtigen Boden 2 des Gehäuses 1 eingeschraubt. Auch hier ist wieder eine sehr flache Kammer 7 gebildet, die gegebenenfalls Leiteinrichtungen enthält, um das Wasser in der Kammer so zu verteilen, dass es überall gleichmäßig aus den Strahlaustrittsöffnungen austritt.

## Patentansprüche

1. Dusche, mit
1.1 einem Brausegehäuse (1),
1.2 einem Wassereinlassanschluss (3, 23),
1.3 einer Strahlscheibe (4), die
1.3.1 mindestens einige, vorzugsweise viele Strahlaustrittsöffnungen aufweist, sowie mit
1.4 einer in dem Brausegehäuse (1) derart angeordneten Beleuchtungseinrichtung (13), dass
1.5 das Licht aus der Strahlscheibe (4) austritt,
wobei
1.6 der Wassereinlass (3, 23) in eine hinter der Strahlscheibe (4) angeordnete flache Kammer (7) führt,
1.7 hinter der die Beleuchtungseinrichtung (10) angeordnet ist und wobei
1.8 die Kammer (7) auf der Seite der Beleuchtungseinrichtung (10) von einer durchsichtigen bis durchscheinenden Wand (2) begrenzt ist und
1.9 die Beleuchtungseinrichtung (10) radial abstrahlende Lichtquellen und eine Umlenkeinrichtung (13) aufweist.

2. Dusche nach Anspruch 1, bei der das Licht nur oder überwiegend aus den Strahlaustrittsöffnungen austritt.

3. Dusche nach Anspruch 1 oder 2, bei der das Licht aus größeren nicht zusammenhängenden Bereichen der Strahlscheibe (4) austritt.

4. Dusche nach einem der vorhergehenden Ansprüche, bei der das Licht im wesentlichen aus der gesamten Fläche der Strahlscheibe (4) austritt.

5. Dusche nach einem der vorhergehenden Ansprüche, mit einer zentralen in der Strahlscheibe (4) angeordneten Lampe.

6. Dusche nach einem der vorhergehenden Ansprüche, bei der die Dicke der Kammer (7) überall etwa gleich ist.

7. Dusche nach einem der vorhergehenden Ansprüche, bei der die Querabmessung der Kammer (7) der der Strahlscheibe (4) etwa gleich ist.

8. Dusche nach einem der vorhergehenden Ansprüche, bei der die Strahlscheibe (4) rund oder quadratisch ist.

9. Dusche nach einem der vorhergehenden Ansprüche, bei der die Kammer (7) und/oder die Strahlscheibe (4) derart ausgebildet ist, dass in ihr Wasserverwirbelungen entstehen.

10. Dusche nach einem der vorhergehenden Ansprüche, bei der die Kammer (7) und/oder die Strahlscheibe (4) derart ausgebildet ist, dass in der Kammer (7) radiale Strömungen entstehen.

11. Dusche nach einem der vorhergehenden Ansprüche, bei der die Beleuchtungseinrichtung (10) LEDs (12) enthält.

12. Dusche nach einem der vorhergehenden Ansprüche, bei der die Beleuchtungseinrichtung (10) Lichtquellen unterschiedlicher Farben aufweist.

13. Dusche nach einem der vorhergehenden Ansprüche, bei der die Brause eine Kopfbrause ist.

14. Dusche nach einem der Ansprüche 1 bis 12, bei der die Brause eine Seitenbrause ist.

15. Dusche nach einem der Ansprüche 1 bis 12, bei der die Brause eine Handbrause ist.

## Claims

1. A shower head comprising
1.1 a housing (1),
1.2 a water-inlet fitting (3, 23),
1.3 a jet-discharge disk (4) having
1.3.1 at least several, preferably numerous, apertures from which jets of water exit, and
1.4 a lighting device (13) arranged in such a manner that in the housing (1)
1.5 light exits from the jet-discharge disk (4)
whereby
1.6 the water inlet (3, 23) leads to a flat chamber (7) arranged upstream of the jet-discharge disk (4).
1.7 behind which the lighting device (10) is arranged and whereby
1.8 the side of the chamber (7) facing the lighting device (10) is bounded by a transparent to translucent wall (2) and
1.9 the lighting device (10) comprises radially emitting light sources and a deflection device (13).

2. A shower head according to claim 1, wherein light exits exclusively, or predominantly, from the apertures of the jet-discharge disk (4).

3. A shower head according to claim 1 or 2, wherein light exits from large, noncontiguous sections of the jet-discharge disk (4).

4. A shower head according to one of the preceding claims, wherein light essentially exits from the entire surface of the jet-discharge disk (4).

5. A shower head according to one of the preceding claims with a lamp centrally arranged in the jet-discharge disk (4).

6. A shower head according to one of the preceding claims , wherein the thickness of the chamber (7) is approximately the same everywhere.

7. A shower head according to one of the preceding claims , wherein the lateral dimension of the chamber (7) is approximately equal to that of the jet-discharge disk (4).

8. A shower head according to one of the preceding claims, wherein the jet-discharge disk (4) is circular or square.

9. A shower head according to one of the preceding claims, wherein the chamber (7) and/or the jet-discharge disk (4) are configured such that hydrodynamic turbulences will occur in the former.

10. A shower head according to one of the preceding claims, wherein the chamber (7) and/or the jet-discharge disk (4) are configured such that radial streamlines occur within the chamber (7).

11. A shower head according to one of the preceding claims, wherein the lighting device (10) contains light emitting diodes (LEDs) (12).

12. A shower head according to one of the preceding claims, wherein the lighting device (10) has light sources emitting light of various colors.

13. A shower head according to one of the preceding claims, wherein the shower head is an overhead shower head.

14. A shower head according to one of claims 1 - 12, wherein the shower head is a side-mounted shower head.

15. A shower head according to one of claims 1-12, wherein the shower head is a hand-held shower head.

## Revendications

1. Douche comprenant
1.1 un boîtier de douchette (1),
1.2 un raccord d'entrée d'eau (3, 23),
1.3 un disque à jets (4) qui
1.31. présente quelques orifices de sortie de jets, de préférence de nombreux orifices de sortie de jets, et comprenant
1.4 un dispositif d'éclairage (13) disposé dans le boîtier de douchette (1) de telle manière que
1.5 la lumière sort du disque à jets (4),
1.6 l'entrée d'eau (3, 23) conduisant dans une chambre (7) plate disposée derrière le disque à jets (4),
1.7 derrière laquelle est disposée le dispositif d'éclairage (10) et
1.8 la chambre (7) étant limitée sur le côté du dispositif d'éclairage (10) par une paroi (2) transparente à translucide et
1.9 le dispositif d'éclairage (10) présentant des sources de lumière rayonnant en direction radiale et un dispositif de renvoi (13).

2. Douche selon la revendication 1, dans laquelle la lumière sort seulement ou principalement des orifices de sortie de jets.

3. Douche selon la revendication 1 ou 2, dans laquelle la lumière sort de grandes parties non communicantes du disque à jets (4).

4. Douche selon l'une quelconque des revendications précédentes, dans laquelle la lumière sort essentiellement de toute la surface du disque à jets (4).

5. Douche selon l'une quelconque des revendications précédentes, comprenant une lampe centrale disposée dans le disque à jets (4).

6. Douche selon l'une quelconque des revendications précédentes, dans laquelle l'épaisseur de la chambre (7) est partout à peu près identique.

7. Douche selon l'une quelconque des revendications précédentes, dans laquelle la dimension transversale de la chambre (7) est à peu près identique à celle du disque à jets (4).

8. Douche selon l'une quelconque des revendications précédentes, dans laquelle le disque à jets (4) est rond ou carré.

9. Douche selon l'une quelconque des revendications précédentes, dans laquelle la chambre (7) et/ou le disque à jets (4) sont exécutés de telle manière que des tourbillons d'eau y sont créés.

10. Douche selon l'une quelconque des revendications précédentes, dans laquelle la chambre (7) et/ou le disque à jets (4) sont exécutés de telle manière que des flux radiaux sont créés dans la chambre (7).

11. Douche selon l'une quelconque des revendications précédentes, dans laquelle le dispositif d'éclairage (10) comprend des LED (12).

12. Douche selon l'une quelconque des revendications précédentes, dans laquelle le dispositif d'éclairage (10) présente des sources de lumière de différentes couleurs.

13. Douche selon l'une quelconque des revendications précédentes, dans laquelle la douche est une douche de tête.

14. Douche selon l'une quelconque des revendications 1 à 12, dans laquelle la douche est une douche latérale.

15. Douche selon l'une quelconque des revendications 1 à 12, dans laquelle la douche est une douchette.
